# EUROPEAN PATENT SPECIFICATION

(11) **EP 4 593 749 B1**
(45) Date of publication and mention of the grant of the patent: **09.12.2026**
(21) Application number: 22793152.4
(22) Date of filing: 26.09.2022
(51) Int. Cl.: A61B 34/20, A61B 90/00, A61B 17/00

(54) **ACCURACY ADJUSTMENT OF NAVIGATED MEDICAL INSTRUMENT**
GENAUIGKEITSEINSTELLUNG EINES NAVIGIERTEN MEDIZINISCHEN INSTRUMENTS
RÉGLAGE DE PRÉCISION D'INSTRUMENT MÉDICAL NAVIGUÉ

(43) Date of publication of application: 06.08.2025
(73) Proprietor: Brainlab SE, 81829 München (DE)
(72) Inventor: ZAUS, Martin, Munich (DE); TRAHANOFSKY, Michael, Munich (DE)
(74) Representative: SSM Sandmair
(86) International application number: PCT/EP2022/076652
(87) International publication number: WO 2024/067944

(56) References cited:
- US-A1- 2005 228 270
- US-A1- 2018 140 223
- US-A1- 2021 236 208
- US-A1- 2022 125 522
- BRAINLAB: "Software User Guide Rev. 1.0 Kolibri cranial/ENT Ver. 2.7", 1 January 2010 (2010-01-01), XP055859399, Retrieved from the Internet <URL:https://userguides.brainlab.com/en/> [retrieved on 20211109]

## Description

### FIELD OF THE INVENTION

The present invention relates to a method for spatially adjusting the locations of centers of markers associated with a medical instrument having a point of interest, a corresponding computer program, a computer-readable storage medium storing such a program and a computer executing the program.

### TECHNICAL BACKGROUND

It is known to navigate medical instruments, like a pointer or a stylus, having a point of interest, like a tip. Those instruments are provided with two or more markers and have a pre-defined geometry, which represents the spatial relationship of the markers and the point of interest. This pre-defined geometry is provided to a medical navigation system which localizes the markers and calculates a calculated location of the point of interest from the locations of the markers and the pre-defined geometry. This assumes that each instrument of a particular design actually has the pre-defined geometry. The present invention aims at providing medical instruments having actual geometries within an acceptable margin around the pre-defined geometry.

In production of medical instruments, deviations can occur between the pre-defined geometry and the actual geometry of a particular instrument. It is thus common to perform quality control. In this quality control, the actual geometry is measured and compared to the pre-defined geometry. In processes so far, an instrument having a deviation above a certain threshold is discarded.

Aspects of the present invention, examples and exemplary steps and their embodiments are disclosed in the following. Different exemplary features of the invention can be combined in accordance with the invention wherever technically expedient and feasible.

Document US 2018/140223 A1 discloses a calibration apparatus for calibrating a medical tool having a tool tracking marker. The calibration apparatus comprises a frame, a frame tracking marker attached to the frame, and a reference point formed on the frame. The reference point provides a known spatial reference point relative to the frame tracking marker.

Document US 2021/236208 A1 discloses a computer implemented medical method of calibrating a medical instrument. The method calculates a position of the instrument tip model within the indentation model, associated with an estimated position of the instrument tip within the indentation and calibrates the medical instrument, thereby using the determined position of the instrument tip model.

Document US 2005/228270 A1 discloses a method and system for improved tracking of an internal object, such as an implant or part of the anatomy, during an image-guided operation. The method may include obtaining a plurality of fiducials for an internal object from a plurality of projection views, obtaining a plurality of measurements for the internal object using the plurality of fiducials, forming a three-dimensional model of the internal object using the plurality of measurements from a plurality of projection views for use in tracking the internal object, and performing an image-guided operation using an image data set and a three-dimensional representation of the internal object.

Document US 2022/125522 A1 discloses devices, systems, and methods for a robotassisted surgery. Navigable instrumentation, which are capable of being navigated by a surgeon using the surgical robot system, and navigation software allow for the navigated placement of interbody fusion devices or other surgical devices.

### EXEMPLARY SHORT DESCRIPTION OF THE INVENTION

In the following, a short description of the specific features of the present invention is given which shall not be understood to limit the invention only to the features or a combination of the features described in this section.

According to the present invention, a medical instrument not passing quality control is not discarded, but rather reworked. In particular, the locations of centers of markers of the medical instrument are adjusted relative to the position of a point of interest (POI) of the medical instrument such that the difference between a calculated location of the POI and the actual location of the POI of a particular medical instrument is within an acceptable tolerance. In this document, the calculated location is calculated from measured locations of the centers of the markers of the instrument and the pre-defined geometry of the medical instrument. The actual location of the POI is typically measured. The scope of the invention is defined solely by the independent claims. Features described in the dependent claims represent optional and preferred embodiments of the invention and may be combined with the independent claims as appropriate.

### GENERAL DESCRIPTION OF THE INVENTION

In this section, a description of the general features of the present invention is given by referring to possible embodiments of the invention.

In general, the invention reaches the aforementioned object by providing, in a first aspect, a method for spatially adjusting the locations of centers of markers associated with a medical instrument having a point of interest. The method comprises executing the following steps.

In a first step, initial locations of the centers relative to the point of interest are measured.

In a second step, corrected locations of the centers relative to the point of interest are calculated.

In a third step, the markers are adjusted such that the locations of the centers of the markers match the corrected locations.

In this document, the term "location" defines where a particular point is in up to three translational dimensions. The combination of a location and a rotational alignment in up to three rotational dimensions is referred to as "position".

Measuring the initial location of the centers of the markers relative to the point of interest is typically performed using a medical tracking system which measures the locations of the centers of the markers in a reference system of the medical tracking system. For reflective markers, the medical tracking system typically comprises a stereoscopic camera, while for electromagnetic markers, the medical tracking system comprises electromagnetic transmitters or receivers.

In order to measure the initial locations of the centers relative to the point of interest, not only the centers of the markers have to be measured, but also the location of the point of interest has to be known.

One option for measuring the location of the point of interest is using a reference tool having a reference point that is adjacent to the point of interest of the medical instrument when the medical instrument is properly positioned on the reference tool. The reference point can be the bottom of a receptacle for accepting the point of interest, such as a tip.

The reference tool can be provided at a known position in the reference system of the medical tracking system, such that the position of the receptacle in the reference system of the medical tracking systems is known. This means that the location of the point of interest of the medical instrument in the reference system of the medical tracking system is also known if it is properly placed in the receptacle. In addition or as an alternative, the reference tool can comprise reference markers which can be detected by the medical tracking system in order to ascertain the position of the reference tool in the reference system of the medical tracking system.

As another option, a navigated pointer can be used to sample the location of the point of interest of the medical instrument. The pointer has a tip and a plurality of pointer markers which can be detected by the medical tracking system. From the known geometry of the pointer and the locations of the pointer markers, the location of the tip of the pointer can be determined, wherein the location of the tip of the pointer then corresponds to the location of the point of interest of the medical instrument.

In a typical application, the medical instrument is positioned in the reference tool, which acts as a cradle for the medical instrument, such that the point of interest of the medical instrument is at the reference point of the reference tool. For example, a tip of the medical instrument is positioned in a recess of the reference tool. The recess preferably has the inverse shape of the tip to accommodate the tip in a pre-defined position. The reference tool is typically positioned such that gravity urges the tip into the recess.

The medical tracking system is then used to determine the locations of the reference markers and of the markers of the medical instrument. From the known geometry of the reference tool, which in particular describes the locations of the reference markers relative to the (bottom of the) recess, the location of the recess in the reference system of the medical tracking system can be calculated. This location is used as the measured, actual location of the point of interest of the medical instrument in the reference system of the medical tracking system. The medical tracking system is further used to measure the initial locations of the centers of the markers in the reference system of the medical tracking system. With this information, the initial locations of the centers relative to the point of interest are known.

In this document, the word "marker" means a marker of the medical instrument. If a marker of another object is meant, a corresponding expression is used. Further, the word "center" generally means the center of a marker. Still further, the expression "point of interest" refers to the point of interest of the medical instrument.

For a perfect medical instrument, the initial locations of the centers relative to the measured location of the point of interest are equal to pre-defined locations of the centers relative to the pre-defined location of the point of interest as represented by the pre-defined geometry. From another point of view, an initial calculated location of the point of interest of the medical instrument can be calculated from the measured initial locations of the centers and the pre-defined geometry of the medical instrument. If the difference between the initial calculated location of the point of interest and the measured location of the point of interest is below a predetermined threshold, the medical instrument is determined to be usable.

However, if this distance is above a predetermined threshold, the medical instrument is determined to be not usable. But instead of discarding such a medical instrument, the medical instrument is reworked to make it usable. In particular, the locations of the centers of the markers of the medical instrument are modified into corrected locations such that a calculated location of the POI, which is calculated from the corrected locations of the centers and the pre-defined geometry, coincides with the actual location of the POI or differs only by the predetermined threshold at most. The actual, measured location of the POI is thus a target location of the POI when calculating the location of the POI.

This reworking of the medical instrument involves calculating the corrected locations of the centers relative to the point of interest. If the markers of the medical instrument are at their corrected locations, applying the pre-defined geometry of the medical instrument for calculating the calculated location of the point of interest of the medical instrument results in a location of the point of interest which corresponds to the target location. Here "corresponds" means an exact match or a match within an allowable tolerance. The corrected locations are for example defined in the reference system of the medical tracking system.

The method therefore further involves adjusting the markers of the medical instrument such that the locations of the centers of the markers match the correct locations. After this adjustment, the pre-defined geometry can be used for the reworked medical instrument to calculate the location of the point of interest from the measured locations of the centers of the markers.

In one embodiment, the markers are flat reflective markers, for example retro-reflective markers. The markers can be stickers which should be attached to the medical instrument at predetermined positions, but manufacturing tolerances might cause deviations from the predetermined positions. In this embodiment, adjusting the markers involves making a part of at least one marker non-reflective. Compared to the initial marker, the adjusted marker has a smaller size. The center of the marker is moved by the adjustment.

By making a part of a flat reflective marker non-reflective, the center of the marker can only be shifted in the plane of the marker. So if all markers of the medical instrument lie in the same plane, or in parallel planes, a relative shift of the markers in a direction perpendicular to this plane is not possible. This can be overcome by providing the flat reflective markers on flat surfaces of the medical instrument which are inclined against a plane in which the centers of the markers lie, and optionally further inclined against each other. Depending on which part of at least one marker is made non-reflective, the relative locations of the centers of the markers of a medical instrument can be adjusted in three dimensions.

In one embodiment, making a part of a marker non-reflective involves applying a non-reflective coating on said part of the marker, removing said part of the marker or inactivating said part of the marker. Applying a non-reflective coating can be made using a jetting technology as it is known from inkjet printers. Removing a part of a marker can be accomplished using radiation, such as laser light, for cutting or burning away said part of the marker. Inactivating a part of a marker means making said part of the marker dull using the radiation.

In one embodiment, adjusting the markers involves modifying the position of at least one marker relative to the medical instrument. This embodiment is suitable for all kinds of markers, such as flat reflective markers, spherical reflective markers and electromagnetic markers. In this embodiment, the size and/or shape of the marker is not modified, but only its position.

In one implementation, modifying the position of a marker relative to the medical instrument involves adjusting of a mechanical adjustment mechanism. The mechanical adjustment mechanism allows a movement of a corresponding marker in at least one of up to three rotational dimensions and/or up to three translational dimensions, for example by adjusting one or more adjustment means, such as an adjusting screw.

In one embodiment, modifying the position of a marker relative to the medical instrument involves adapting the position of said markers and hardening a connection of said marker to the medical instrument. There are numerous exemplary implementations within this embodiment.

In one implementation, said marker is attached to a holder such as a rod, having a loose end which is inserted into soft glue or any soft material which can be hardened once the marker is in its desired position. The soft medium that hardens during this procedure refers to the connection of the present implementation. The marker can be brought into the corrected position for example using a mechanical adjustment mechanism as explained above or other means, such as a robotic arm. However, once the connection is hardened, the adjustment mechanism can be removed.

In another implementation, the connection of the marker to the medical instrument is first softened, for example by applying heat, before the position is modified and the connection hardens again.

**In** one embodiment, calculating the corrected locations of the centers involves finding optimized locations of the centers for which the distance between a calculated location of the point of interest of the medical instrument and the target location of the point of interest of the medical instrument is minimized. It shall be noted that the locations do not necessarily have to be absolutely optimized, but might be optimized within a certain granularity within the optimization, such as a granularity of a tenth of a millimeter, a hundredth of a millimeter or a thousandth of a millimeter.

In this embodiment, the calculated location of the point of interest is calculated from a set of candidate locations of the centers and generic calibration data representing the pre-defined geometry of the medical instrument. The set of candidate locations comprises one candidate location for each marker of the medical instrument. The candidate locations are assumed locations, or locations under test, for which the calculated location of a point of interest is calculated using the pre-defined geometry of the medical instrument which is to be met by the medical instrument. In other words, the present embodiment finds those candidate locations as the optimized locations of the centers of the markers such that the calculated location of the point of interest, when calculated from the set of candidate locations of the centers, coincides with the target location of the point of interest. Once again, a small deviation might be acceptable.

In one implementation, the pre-defined geometry of the medical instrument represents pre-defined locations of the centers of the markers and of the point of interest of an ideal medical instrument. Calculating the calculated location of the point of interest involves transforming the pre-defined geometry such that the pre-defined locations of the centers are aligned with the candidate locations of the centers and using the location of the point of interest in the transformed pre-defined geometry as the calculated location of the point of interest. In other words, the pre-defined geometry of the medical instrument is transformed such that the locations of the centers of the markers of the pre-defined geometry match the measured locations of the centers of the markers. This for example involves a rigid matching in which the transformation of the pre-defined geometry is limited to a uniform scaling of the pre-defined geometry in three orthogonal dimensions, a rotation of the pre-defined geometry in up to three rotational dimensions and a translation of the scaled and rotated predefined geometry in up to three orthogonal translational dimensions. When the markers in the transformed pre-defined geometry match the measured markers, the location of the point of interest in the transformed pre-defined geometry is the calculated location of the point of interest.

In one embodiment, calculating the corrected locations of the centers involves calculating multiple calculated locations of the point of interest, one calculated location for each one of multiple sets of candidate locations of the centers. The candidate locations resulting in the smallest distance between the corresponding calculated location of the point of interest and the target location of the point of interest are selected as the calculated locations of the centers.

In general, this process can follow a brute force approach in which each of a plurality of sets of candidate locations is tested. However, it is preferable to use an appropriate optimization approach which is computationally less expensive.

In one implementation, the multiple sets of candidate locations are selected from a search space which is limited to achievable locations of the centers. Limiting the search space reduces the computational complexity since candidate locations which are impossible to be achieved do not have to be tested.

In one implementation, the achievable locations of the centers are determined by the properties of the corresponding markers. For spherical reflective markers or electromagnetic markers, the properties for example describe the range in which the location of the marker can be adjusted. This range for example depends on the properties of the mechanical adjustment mechanism or the range in which the connection of the marker to the medical instrument can be set before the connection is hardened.

If the marker is a flat reflective marker, the properties can be the minimum marker size and/or the possible location of the center of the markers. The minimum size is the size required for the medical tracking system to reliably localize the marker and can, for example, be 1 cm², 1.5 cm² or 2 cm². If adjusting the marker involves making a part of the marker non-reflective, this means that the area or reflective rest of the adjusted marker must lie within the area of the marker before adjustment. For a circular reflective marker, the minimum size means that its center cannot be moved closer to the boundary of the marker before its adjustment than the required minimum radius of the marker after adjustment.

In one embodiment, the pre-defined geometry of the medical instrument represents pre-defined locations of the centers of markers and of a point of interest and calculating the calculated location of the point of interest involves generating a plurality of transformation matrices comprising a transformation matrix for each marker. Each transformation matrix represents the transformation of the pre-defined location of the center of the marker into the pre-defined location of the point of interest. The transformation matrix describes the spatial relation of the pre-defined location of the center of the marker to the pre-defined location of the point of interest. The result of a multiplication of the transformation matrix with the location of the center of a marker thus is the pre-defined location of the point of interest.

Calculating the calculated location of the point of interest further involves assembling the plurality of transformation matrices into an overall transformation matrix and multiplying the overall transformation matrix by a candidate matrix comprising the set of candidate locations. The result of this multiplication is a vector representing the calculated location of the point of interest for the set of candidate locations.

The advantage of this embodiment is that the overall transformation matrix only has to be calculated once since it describes the pre-defined geometry of the medical instrument. Calculating the calculated location of the point of interest is then a simple matrix multiplication, which involves a low computational complexity.

The arrangement of the plurality of transformation matrices in the overall transformation matrix and the arrangement of the candidate locations in the candidate matrix is left to the particular implementation as long as it is consistent.

**In** a second aspect, the invention is directed to a computer program comprising instructions which, when the program is executed by at least one computer, causes the at least one computer to acquire measurement data of a medical instrument having a plurality of markers and a point of interest, the measurement data representing initial locations of centers of the markers relative to the point of interest, and to calculate correction data representing corrected locations of the centers relative to the point of interest.

The computer program thus relates to the parts of the method according to the first aspect which can be performed by a computer. This involves all such parts of the first aspects.

**In** one embodiment, the program causes the computer to further output modification data indicating how the markers have to be adapted such that the locations of the centers of the markers match the corrected locations. The output modification data can be output to a manipulation device, such as a laser treating a part of at least one marker.

The output modification data for example defines the part of the marker to be made non-reflective, for example as an area defined in the reference system of the medical navigation system. In another example, the output modification data describe how adjustment means of a mechanical adjustment mechanism have to be manipulated, for example in terms of the number of turns of an adjustment screw.

The invention may alternatively or additionally relate to a (physical, for example electrical, for example technically generated) signal wave, for example a digital signal wave, such as an electromagnetic carrier wave carrying information which represents the program, for example the aforementioned program, which for example comprises code means which are adapted to perform any or all of the computer-executable steps of the method according to the first aspect. The signal wave is in one example a data carrier signal carrying the aforementioned computer program. A computer program stored on a disc is a data file, and when the file is read out and transmitted it becomes a data stream for example in the form of a (physical, for example electrical, for example technically generated) signal. The signal can be implemented as the signal wave, for example as the electromagnetic carrier wave which is described herein. For example, the signal, for example the signal wave is constituted to be transmitted via a computer network, for example LAN, WLAN, WAN, mobile network, for example the internet. For example, the signal, for example the signal wave, is constituted to be transmitted by optic or acoustic data transmission. The invention according to the second aspect therefore may alternatively or additionally relate to a data stream representative of the aforementioned program, i.e. comprising the program.

In a third aspect, the invention is directed to a computer-readable storage medium on which the program according to the second aspect is stored. The program storage medium is for example non-transitory.

In a fourth aspect, the invention is directed to at least one computer (for example, a computer), comprising at least one processor (for example, a processor), wherein the program according to the second aspect is executed by the processor, or wherein the at least one computer comprises the computer-readable storage medium according to the third aspect.

In a fifth aspect, the disclosure is directed to a system, comprising: a reference tool, a tracking system and a computer according to the fourth aspect and connected to the tracking system. The computer localizes the markers on the reference tool and the markers on the instrument placed on the reference tool via the tracking system and calculates correction data representing corrected locations of the centers of the markers on the instrument relative to the point of interest.

In one embodiment, the system further comprises a laser for removing a part of at least one marker or a spraying device for spraying a liquid on a part of at least one marker, both in accordance with the correction data calculated by the computer.

Alternatively or additionally, the invention according to the fifth aspect is directed to a for example non-transitory computer-readable program storage medium storing a program for causing the computer according to the fourth aspect to execute the computer-executable steps of the method according to the first aspect.

### DEFINITIONS

In this section, definitions for specific terminology used in this disclosure are offered which also form part of the present disclosure.

### Marker

It is the function of a marker to be detected by a marker detection device (for example, a camera or an ultrasound receiver or analytical devices such as CT or MRI devices) in such a way that its spatial position (i.e. its spatial location and/or alignment) can be ascertained. The detection device is for example part of a navigation system. The markers can be active markers. An active marker can for example emit electromagnetic radiation and/or waves which can be in the infrared, visible and/or ultraviolet spectral range. A marker can also however be passive, i.e. can for example reflect electromagnetic radiation in the infrared, visible and/or ultraviolet spectral range or can block x-ray radiation. To this end, the marker can be provided with a surface which has corresponding reflective properties or can be made of metal in order to block the x-ray radiation. It is also possible for a marker to reflect and/or emit electromagnetic radiation and/or waves in the radio frequency range or at ultrasound wavelengths. A marker preferably has a spherical and/or spheroid shape and can therefore be referred to as a marker sphere; markers can however also exhibit a cornered, for example cubic, shape.

### Pointer

A pointer is a rod which comprises one or more - advantageously, two - markers fastened to it and which can be used to measure off individual co-ordinates, for example spatial co-ordinates (i.e. three-dimensional co-ordinates), on a part of the body, wherein a user guides the pointer (for example, a part of the pointer which has a defined and advantageously fixed position with respect to the at least one marker attached to the pointer) to the position corresponding to the co-ordinates, such that the position of the pointer can be determined by using a surgical navigation system to detect the marker on the pointer. The relative location between the markers of the pointer and the part of the pointer used to measure off co-ordinates (for example, the tip of the pointer) is for example known. The surgical navigation system then enables the location (of the three-dimensional co-ordinates) to be assigned to a predetermined body structure, wherein the assignment can be made automatically or by user intervention.

### Medical tracking system

A tracking system, such as a medical tracking system, is understood to mean a system which can comprise: at least one marker device; a transmitter which emits electromagnetic waves and/or radiation and/or ultrasound waves; a receiver which receives electromagnetic waves and/or radiation and/or ultrasound waves; and an electronic data processing device which is connected to the receiver and/or the transmitter, wherein the data processing device (for example, a computer) for example comprises a processor (CPU) and a working memory, wherein electronic data processing device processes the output of the receiver to determine the location of the at least one marker device, for example in a reference system of the tracking system.

### BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein
- Fig. 1: shows a partly exploded view of a medical instrument;
- Fig. 2: shows steps of a method according to the present invention;
- Fig. 3: shows the medical instrument in a reference tool;
- Fig. 4: shows a brute force optimization approach;
- Fig. 5: shows an exemplary marker adjustment; and

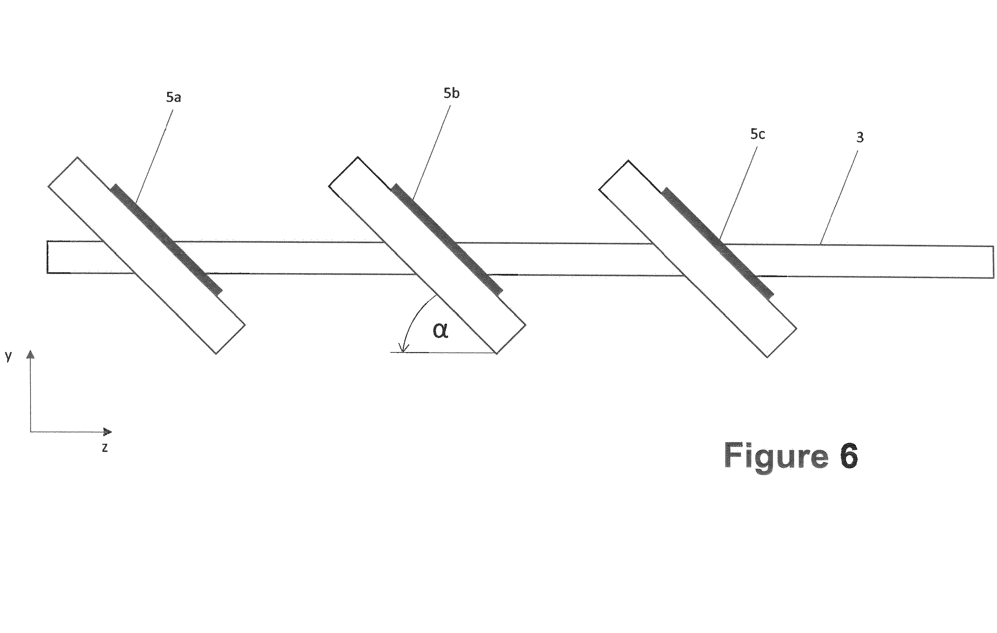

- Fig. 6: shows marker adjustment in three dimensions.

### DESCRIPTION OF EMBODIMENTS

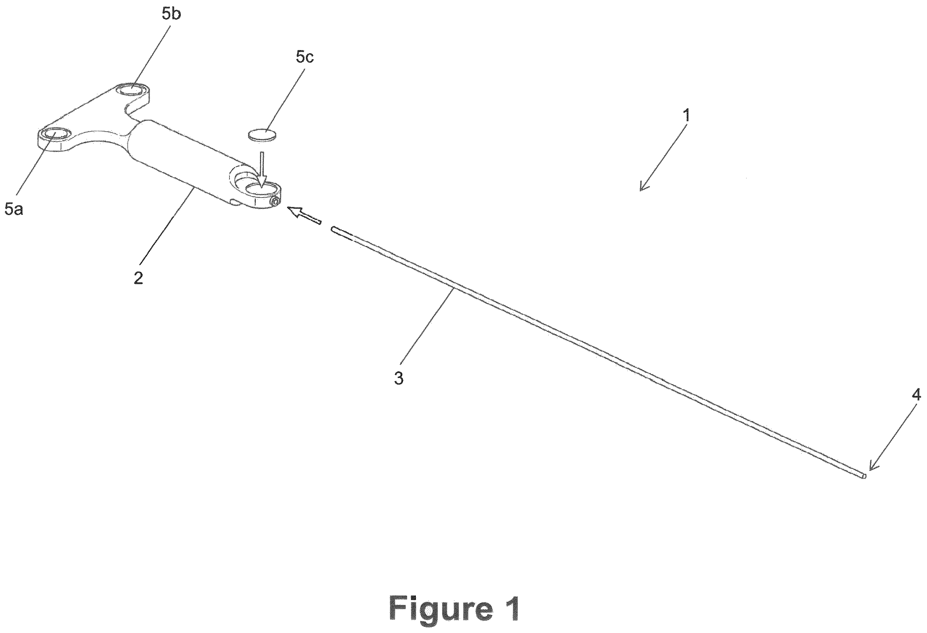

Figure 1 shows a schematic view of a medical instrument 1. The medical instrument 1 has a body 2 made of plastic and carrying three flat reflective markers 5a, 5b and 5c. In the illustration of Figure 1, the marker 5c is not attached to the body 2 yet.

The medical instrument 1 further has a rod 3 made of metal and attached to the body 2. At the end of the rod 3 which is distant from the body 2, the rod 3 has a tip 4, which is the point of interest of the medical instrument 1.

The medical instrument 1 of this exemplary embodiment is a disposable stylet which is manufactured in large numbers. The medical instrument 1 is to be tracked using a medical tracking system, which means that the locations of the centers of the markers 5 are detected and the location of the tip 4 is calculated from the detected locations of the centers of the markers 5. This requires that each medical instrument of this design has the same geometry, namely a pre-defined geometry known to the medical tracking system. By applying the pre-defined geometry to the measured locations of the centers of the markers 5, the medical tracking system can calculate the location of the tip 4.

However, the medical instrument 1 might not have the pre-defined geometry for several reasons. The first reason is that one or more of the markers 5 are not at the desired location on the body 2. Another reason is that the rod 3 is not straight. Yet another reason is that the rod 3 is not properly assembled to the body 2. Any imperfection of the actual medical instrument 1 can cause a deviation of the actual position of the tip 4 from a calculated position of the tip 4 which is calculated from the measured locations of the markers 5 and the pre-defined geometry of the medical instrument.

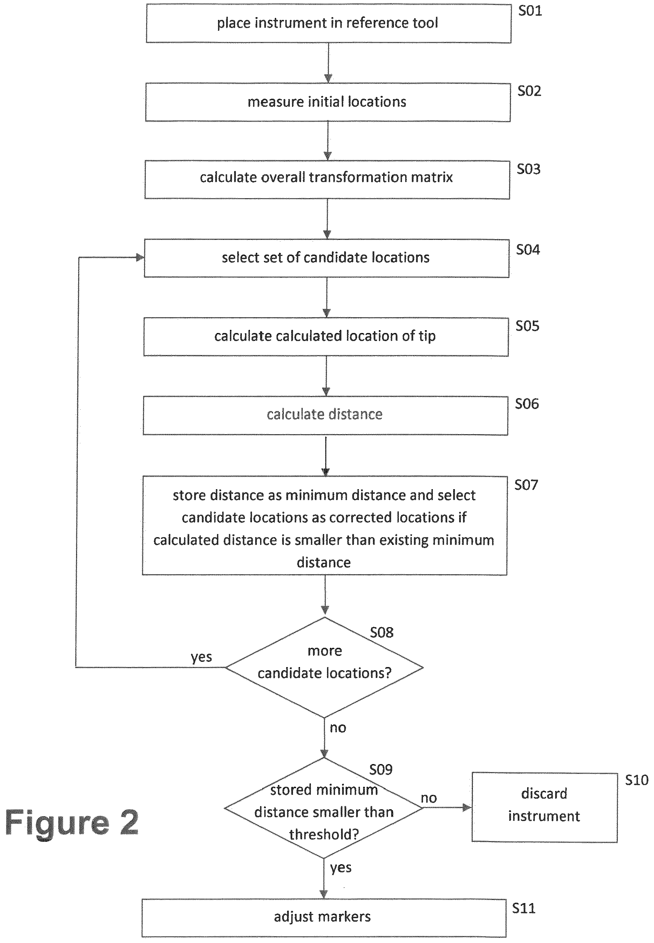

The flow diagram of Figure 2 shows exemplary steps of a method for spatially adjusting the locations of centers of the markers 5 associated with the medical instrument 1 having a point of interest in terms of the tip 4.

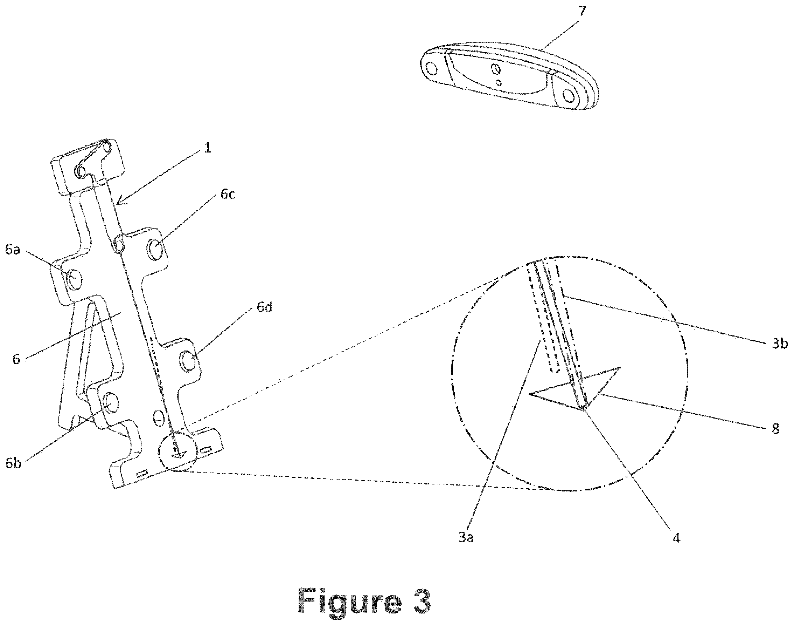

At step S01, the medical instrument 1 is placed in a reference tool 6 as shown in Figure 3. The reference tool 6 has markers 6a, 6b, 6c and 6d and a recess 8 for receiving the tip 4 of the medical instrument 1. The bottom of the recess 5 is a reference location for the tip 4 at a predetermined location of the reference tool 6. The geometry of the reference tool 6, which defines the locations of the markers 6a to 6d and the recess 8 relative to each other, is known to the medical tracking system.

Step S02 involves measuring the initial locations of the centers of the markers 5 relative to the tip 4 using the medical tracking system.

The camera 7 of the medical tracking system captures two images which show each marker from slightly different viewing directions. The medical tracking system analyses the images and thus determines the location of the center of a marker in a reference system of the medical tracking system, which is for example associated with the camera 7.

The medical tracking system measures the initial locations of the centers of the markers 5. It further measures the locations of the reference markers 6a to 6d. From the locations of the reference markers 6a to 6d and the known geometry of the reference tool 6, the medical tracking system derives the location of the reference location at the bottom of the recess 8, and thus the actual location of the tip 4. The medical tracking system therefore measures the initial locations of the centers of the markers 5 relative to the tip 4. The actual location of the tip 4 is used as a target location of the tip 4 in the subsequent calculation of corrected locations of the centers of the markers 5.

The measured initial location of a center of a marker is represented by a vector *x*_{*m*,*i*} = (*t_{mi,x}, t_{mi,y}, t_{mi,z}*)*^{T}*, wherein t_{mi,x}, t_{mi,y} and t_{mi,z} are the measured coordinates of the center of the marker i in the x, y and z dimension, respectively. Here, i is the index indicating a marker 5 of the medical instrument, with i = 1, ..., n, where n is the total number of markers of the instrument. In the present example, n = 3.

The enlarged part of Figure 3 does not only show the tip 4 in the recess 8, but also an initial calculated location of the tip 4 indicated by the dashed outline 3a of the rod 3. The initial calculated location of the tip 4 is calculated from the initial locations of the centers of the markers 5 and the pre-defined geometry of the medical instrument 1.

When the pre-defined geometry of the medical instrument 1 is applied to the initial locations of the centers of the markers 5, the initial calculated location of the tip 4 deviates from the actual, measured location of the tip 4.

Step S03 involves calculating an overall transformation matrix ***C̃*** that describes how pre-defined locations of the centers of the markers 5 transform into the pre-defined location of the tip 4 for the pre-defined geometry of the medical instrument 1.

The locations of the centers of the markers according to the pre-defined geometry are given as ${\overline{x}}_{i} = {\left[{x}_{i}^{T} 1\right]}^{T}$ with *xᵢ* = [*tᵢₓ t_{iy} t_{iz}*]*^{T},* wherein tᵢₓ, t_{iy} and t_{iz} are the coordinates of the center of the marker i in the x, y and z dimension, respectively. Here, i is the index indicating a marker 5 of the medical instrument, with i = 1,...,n, where n is the total number of markers of the instrument. In the present example, n = 3.

Each marker 5 has an associated transformation matrix Mᵢ that describes how the location of the center of the corresponding marker transforms into the location of the center of the tip 4. The general form is ${T}_{i} = \left[\begin{matrix}{R}_{i} & {t}_{i} \\ \overline{0} & 1\end{matrix}\right] ,$ wherein Rᵢ is a rotation matrix of size 3x3, tᵢ is a translation vector of size 3x1, 0̅ is a zero vector of size 1x3 and 1 is a scalar. With this, ***Mᵢ*** = ***ΠTᵢ*** since only the location of the tip 4 is relevant, with $Π = \left[\begin{matrix}1 & 0 & 0 & 0 \\ 0 & 1 & 0 & 0 \\ 0 & 0 & 1 & 0\end{matrix}\right] .$

The overall transformation matrix is then ***C̃*** = **[*M*₁ *M*₂** ... ***Mₙ*].**

Step S04 involves selecting a set of candidate locations of the centers of the markers 5. The set of candidate locations comprises one candidate location for each of the markers 5a, 5b and 5c. The set of candidate locations is selected from a search space which is limited to achievable locations of the centers of the markers 5. The center of an adjusted marker 5 must lie within the surface of the initial marker since the reflective part of the marker can only be made smaller. The center is further limited by the fact that a marker after adjustment must have a particular minimum size.

The set of candidate locations is represented by a vector ${X}_{c} = \left[\begin{matrix}{x}_{c , 1} \\ {x}_{c , 2} \\ ⋯ \\ {x}_{c , n}\end{matrix}\right]$ with ${X}_{c , i} = \left[\begin{matrix}{t}_{ci , x} \\ {t}_{ci , y} \\ {t}_{ci , z} \\ 1\end{matrix}\right] .$

Here, t_{ci,x}, t_{ci,y} and t_{ci,z} are the coordinates of a candidate location of the marker having the index i, with i = 1, 2, ..., n and n being the number of markers of the medical instrument.

Step S05 involves calculating a calculated location of the tip 4 for the selected set of candidate locations of the centers of the markers 5 using the overall transformation matrix as *x_{P} =* (*tₚₓ, t_{py}, t_{pz}*)*^{T}* = ***C̃X_{c}.*** The calculated location of the tip 4 is calculated in the reference system of the medical tracking system.

Step S06 involves calculating the distance between the target location of the tip 4 and the calculated location xₚ of the tip 4. This distance is for example the Euclidian distance between the calculated location and the target location.

Step S07 involves storing the distance calculated in step S06 as a minimum distance if the distance calculated in step S06 is smaller than the hitherto stored minimum distance. In this case, the candidate locations of the set of candidate locations selected in step S04 are stored as the corrected locations of the centers of the markers 5. In the first iteration of the loop, the distance calculated in step S06 is automatically set as the minimum distance.

Step S08 involves determining whether there is at least one set of candidate locations in the search space which has not been investigated yet. If this is the case, the flow chart returns to step S04 where a hitherto untested set of candidate locations is selected and steps S05 to S08 are repeated with the new set of candidate locations.

The search space is for example defined around the initial locations of the centers of the markers, in particular centered around the initial locations.

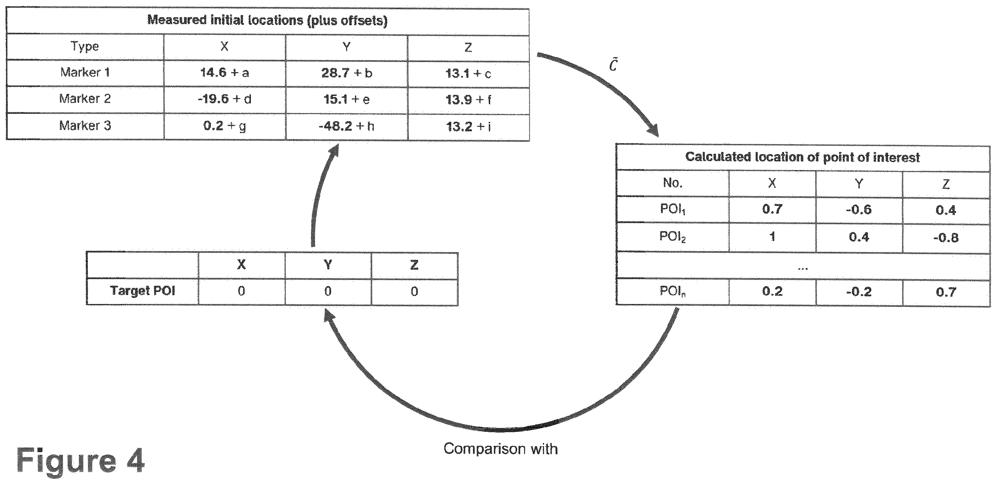

Figure 4 shows a table with exemplary initial locations of the centers of the markers 5a, 5b and 5c. In the process described above, each coordinate is varied using an offset a to i, thus resulting in a search space having nine dimensions. In a brute force approach, the search space is processed with a predetermined granularity of the offsets to test a number of sets of candidate locations. Each set of candidate locations results in a calculated location of the tip 4, given as POI₁ to POIₙ in the right table of Figure 4. The candidate locations in the set of candidate locations resulting in a calculated position of the tip 4 which is closest to the target location of the tip 4 are selected as the corrected locations. In Figure 4, the target location is referred to as target POI.

If it is determined in step S08 that all sets of candidate locations in the search space have been investigated, the flow diagram proceeds to step S09. In this step, it is determined whether or not the minimum distance is equal to or smaller than a threshold. If this is not the case, this means that the markers 5 of the medical instrument 1 cannot be adjusted to make the medical instrument 1 acceptable for use. The flow diagram thus proceeds with step S10 where the medical instrument 1 is discarded.

However, if the minimum distance is equal to or smaller than the threshold, the flow diagram proceeds to step S11 where the markers 5 are adjusted such that their centers have the corrected locations.

The dot and dash line 3b in Figure 3 shows a part of the rod 3 as it would be located if the locations of the centers of the markers 5 would be measured again after the adjustment of the markers 5 and the pre-determined geometry of the medical instrument 1 would be applied.

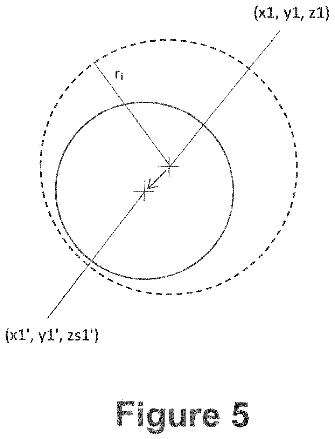

**In** the present example of flat reflective markers having a circular shape, a laser (not shown) is used to make a part of a marker non-reflective as shown in Figure 5.

The dashed circle in Figure 5 shows the initial marker having an initial center at a position (x1, y1, z1). The full circle of Figure 5 shows the marker after adjustment, where the area between the full circle and the dashed circle was made dull or removed using the laser. The new location of the center of the marker is now (x1', y1', z1'). The arrow indicates the shift of the center of the marker by the adjustment.

Rather than using a brute force approach, a numerical optimization can be performed. The offsets a to i shown in Figure 4, which are added to the initial locations of the centers of the markers to form the candidate locations, are arranged in a vector ***θ̅*** = **(*a, b, c,* ... , *i*)*^{T}.*** The optimization to perform is then ${argmin}_{θ} \left[{β}_{1}\left({C}^{˜}∗θ-\hat{P}\right)+{β}_{2}{\left‖θ\right‖}_{2}\right] ,$ wherein β₁ and β₂ are scalars to weight the two terms in the argument. The second term represents the deviation of the candidate locations from the initial locations to favor small deviations. ***P̂*** is the vector from the reference location of the reference tool 6, and thus of the target location of the tip 4, to the calculated location of the tip 4 calculated from the initial locations of the centers of the markers 5 and the pre-defined geometry of the medical instrument 1.

In figure 5, rᵢ is the initial radius of the marker. The new center of the marker must lie within the initial area of the marker, which means a constraint for the corrected location of the center of the marker. In addition, the adjusted marker must have a minimum radius rₘᵢₙ in order to maintain detectable by the medical tracking system. This means a constraint for a particular marker expressed as ${θ}_{x}^{2} + {θ}_{y}^{2} + {θ}_{z}^{2} \leq {\left({r}_{i}-{r}_{min}\right)}^{2} ,$ wherein θₓ, θ_{y} and θ_{z} are the offsets of the x, y and z coordinates, respectively, of the center of a particular marker between the initial location and the corrected location. This restriction applies to each of the markers, resulting in an overall restriction for the vector ***θ̅*** when the restrictions for all markers are aggregated.

In the exemplary medical instrument 1 shown in Figure 1, all three markers 5a, 5b and 5c are flat markers lying in the same plane. It is therefore not possible to adjust the centers of the markers 5 to compensate for a deviation of the location of the tip 4 in a direction perpendicular to this plane.

Figure 7 shows an exemplary arrangement in which the markers are arranged on inclined surfaces such that the locations of the centers of the markers relative to each other can be adjusted in three orthogonal translational dimensions. With this arrangement, the search space for each candidate location of a marker is defined by the orientation of the plane in which the corresponding marker lies, a minimum size of the corresponding marker and the initial location of the center of the corresponding marker.

In Figure 7, the y and z axes span the drawing plane and the x axis is perpendicular to the drawing plane. The markers 5 lie in a plane which is perpendicular to the drawing plane and inclined relative to the xy plane and the xz plane. This means that the center of a marker 5 can be shifted independently along the x axis. However, a shift along the y axis implies a shift in the z axis and vice versa. This means a restriction for a particular marker expressed as $a {θ}_{z}^{2} + b {θ}_{y}^{2} = {c}^{2} ,$ wherein a, b and c are scalars that depend on the orientation of the plane of the marker. θ_{y} and θ_{z} are the offsets of the y and z coordinates, respectively, of the center of the marker between the initial location and the corrected location. This restriction applies to each of the markers 5, which leads to an overall restriction for the vector ***θ̅*** when the restrictions for all markers are aggregated.

## Claims

1. A method for spatially adjusting the locations of centers of markers (5) associated with a medical instrument (1) having a point of interest (4), the method comprising the steps of:
- measuring initial locations of the centers relative to the point of interest (4),
- calculating corrected locations of the centers relative to the point of interest (4) based on a pre-defined geometry of the medical instrument (1), and
- adjusting the markers (5) such that the locations of the centers of the markers (5) match the corrected locations.

2. The method of claim 1, wherein the markers (5) are flat reflective markers and adjusting the markers (5) involves making a part of at least one marker (5) non-reflective.

3. The method of claim 2, wherein making a part of a marker (5) non-reflective involves applying a non-reflective coating on said part of the marker (5), removing said part of the marker (5) or inactivating said part of the marker (5).

4. The method of any one of claims 1 to 3, wherein adjusting the markers (5) involves modifying the position of at least one marker (5) relative to the medical instrument (1).

5. The method of claim 4, wherein modifying the position of a marker (5) relative to the medical instrument involves adjusting of a mechanical adjustment mechanism.

6. The method of any one of claims 4 or 5, wherein modifying the position of a marker (5) relative to the medical instrument (1) involves adapting the position of said marker (1) and hardening a connection of said marker (5) to the medical instrument (1).

7. The method of any one of claims 1 to 6, wherein calculating the corrected locations of the centers involves finding optimized locations of the centers for which the distance between a calculated location of the point of interest (4) of the medical instrument (1) and a target location of the point of interest (4) of the medical instrument (1) is minimized, wherein the calculated location of the point of interest (4) is calculated from a set of candidate locations of the centers and generic calibration data representing the pre-defined geometry of the medical instrument (1).

8. The method of claim 7, wherein the pre-defined geometry of the medical instrument (1) represents pre-defined locations of the centers of markers (5) and of a point of interest (4) and calculating the calculated location of the point of interest (4) involves transforming the pre-defined geometry such that the pre-defined locations of the centers are aligned with the candidate locations of the centers and using the location of the point of interest (4) in the transformed pre-defined geometry as the calculated location of the point of interest (4).

9. The method of claim 7 or 8, wherein calculating the corrected locations of the centers involves calculating multiple calculated locations of the point of interest (4) for multiple sets of candidate locations of the centers and the candidate locations resulting in the smallest distance between the corresponding calculated location of the point of interest (4) and the target location of the point of interest (4) are selected as the calculated locations of the centers.

10. The method of claim 9, wherein the multiple sets of candidate locations are selected from a search space which is limited to achievable locations of the centers.

11. The method of claim 7, the pre-defined geometry of the medical instrument (1) represents pre-defined locations of the centers of markers (5) and of a point of interest (4) and calculating the calculated location of the point of interest (4) involves:
- generating a plurality of transformation matrices (Mᵢ), comprising a transformation matrix for each marker (5), a transformation matrix representing the transformation of the pre-defined location of the center of the marker (5) into the predefined location of the point of interest (4),
- assembling an overall transformation matrix (*C̃*) from the plurality of transformation matrices (Mᵢ), and
- multiplying the transformation matrix (*C̃*) by a candidate vector (X_{c}) comprising the set of candidate locations.

12. A computer program which, when running on a computer, causes the computer to:
- acquire measurement data of a medical instrument (1) having a plurality of markers (5) and a point of interest (4), the measurement data representing initial locations of centers of the markers (5) relative to the point of interest (4), and
- calculate correction data representing corrected locations of the centers relative to the point of interest (4) based on a pre-defined geometry of the medical instrument (1).

13. The computer program of claim 12, wherein the program causes the computer to further output modification data indicating how the markers (5) have to be adapted such that the locations of the centers of the markers (5) match the corrected locations.

14. A computer on which the computer program according to claim 12 or 13 is stored and/or run.

15. A non-transitory computer readable storage medium on which the program according to claim 12 or 13 is stored.

## Patentansprüche

1. Ein Verfahren zur räumlichen Anpassung der Positionen von Markerzentren (5), die einem medizinischen Instrument (1) mit einem Punkt von Interesse (4) zugeordnet sind, wobei das Verfahren die folgenden Schritte umfasst:
- Messen der initialen Positionen der Mittelpunkte relativ zum Punkt von Interesse (4),
- Berechnen korrigierter Positionen der Mittelpunkte relativ zum Punkt von Interesse (4) auf der Grundlage einer vordefinierten Geometrie des medizinischen Instruments (1) und
- Anpassen der Marker (5) derart, dass die Positionen der Mittelpunkte der Marker (5) mit den korrigierten Positionen übereinstimmen.

2. Das Verfahren nach Anspruch 1, wobei die Marker (5) flache reflektierende Marker sind und das Anpassen der Marker (5) darin besteht, einen Teil mindestens eines Markers (5) nicht reflektierend zu machen.

3. Das Verfahren nach Anspruch 2, wobei das Nichtreflektierendmachen eines Teils eines Markers (5) das Aufbringen einer nichtreflektierenden Beschichtung auf diesen Teil des Markers (5), das Entfernen dieses Teils des Markers (5) oder das Inaktivieren dieses Teils des Markers (5) umfasst.

4. Das Verfahren nach einem der Ansprüche 1 bis 3, wobei das Anpassen der Markierungen (5) das Verändern der Position mindestens einer Markierung (5) relativ zum medizinischen Instrument (1) umfasst.

5. Das Verfahren nach Anspruch 4, wobei das Verändern der Position eines Markers (5) relativ zum medizinischen Instrument das Einstellen eines mechanischen Einstellmechanismus umfasst.

6. Das Verfahren nach einem der Ansprüche 4 oder 5, wobei das Verändern der Position eines Markers (5) relativ zum medizinischen Instrument (1) das Anpassen der Position des Markers (5) und das Verfestigen einer Verbindung des Markers (5) mit dem medizinischen Instrument (1) umfasst.

7. Das Verfahren nach einem der Ansprüche 1 bis 6, wobei das Berechnen der korrigierten Positionen der Mittelpunkte das Ermitteln optimierter Positionen der Mittelpunkte umfasst, für die der Abstand zwischen einer berechneten Position des Punktes von Interesse (4) des medizinischen Instruments (1) und einer Zielposition des Punktes von Interesse (4) des medizinischen Instruments (1) minimiert wird, wobei die berechnete Position des Punktes von Interesse (4) aus einer Menge von Kandidatenpositionen der Mittelpunkte und generischen Kalibrierungsdaten berechnet wird, die die vordefinierte Geometrie des medizinischen Instruments (1) darstellen.

8. Das Verfahren nach Anspruch 7, wobei die vordefinierte Geometrie des medizinischen Instruments (1) vordefinierte Positionen der Mittelpunkte von Markern (5) sowie eines Punktes von Interesse (4) darstellt und die Berechnung der berechneten Position des interessierenden Punktes (4) die Transformation der vordefinierten Geometrie derart umfasst, dass die vordefinierten Positionen der Mittelpunkte mit den Kandidatenpositionen der Mittelpunkte ausgerichtet werden, sowie die Verwendung der Position des Punktes von Interesse (4) in der transformierten vordefinierten Geometrie als berechnete Position des Punktes von Interesse (4).

9. Das Verfahren nach Anspruch 7 oder 8, wobei das Berechnen der korrigierten Positionen der Mittelpunkte das Berechnen mehrerer berechneter Positionen des Punktes von Interesse (4) für mehrere Sätze von Kandidatenpositionen der Mittelpunkte umfasst und die Kandidatenpositionen, die den kleinsten Abstand zwischen der entsprechenden berechneten Position des Punktes von Interesse (4) und der Zielposition des Punktes von Interesse (4) ergeben, als die berechneten Positionen der Mittelpunkte ausgewählt werden.

10. Das Verfahren nach Anspruch 9, wobei die mehreren Sätze von Kandidatenpositionen aus einem Suchraum ausgewählt werden, der auf erreichbare Positionen der Mittelpunkte beschränkt ist.

11. Das Verfahren nach Anspruch 7, wobei die vordefinierte Geometrie des medizinischen Instruments (1) vordefinierte Positionen der Mittelpunkte von Markern (5) und eines Punktes von Interesse (4) darstellt und die Berechnung der berechneten Position des Punktes von Interesse (4) Folgendes umfasst:
- Erzeugen einer Vielzahl von Transformationsmatrizen (Mᵢ), umfassend eine Transformationsmatrix für jeden Marker (5), wobei jede Transformationsmatrix die Transformation der vordefinierten Position des Mittelpunkts des Markers (5) in die vordefinierte Position des Punktes von Interesse (4) darstellt,
- Zusammenstellen einer Gesamttransformationsmatrix (*C̃*) aus der Vielzahl von Transformationsmatrizen (Mᵢ) und
- Multiplizieren der Transformationsmatrix (*C̃*) mit einem Kandidatenvektor (X_{c}), der die Menge der Kandidatenpositionen umfasst.

12. Ein Computerprogramm, das, wenn es auf einem Computer ausgeführt wird, den Computer dazu veranlasst:
- Messdaten eines medizinischen Instruments (1) mit einer Vielzahl von Markern (5) und einem Punkt von Interesse (4) zu erfassen, wobei die Messdaten die anfänglichen Positionen der Mittelpunkte der Marker (5) relativ zum Punkt von Interesse (4) darstellen, und
- Korrekturdaten berechnet, die korrigierte Positionen der Mittelpunkte relativ zum Punkt von Interesse (4) darstellen, basierend auf einer vordefinierten Geometrie des medizinischen Instruments (1).

13. Das Computerprogramm nach Anspruch 12, wobei das Programm den Computer veranlasst, ferner Modifikationsdaten auszugeben, die angeben, wie die Marker (5) angepasst werden müssen, damit die Positionen der Mittelpunkte der Marker (5) mit den korrigierten Positionen übereinstimmen.

14. Ein Computer, auf dem das Computerprogramm gemäß Anspruch 12 oder 13 gespeichert ist und/oder ausgeführt wird.

15. Ein nichtflüchtiges, computerlesbares Speichermedium, auf dem das Programm gemäß Anspruch 12 oder 13 gespeichert ist.

## Revendications

1. Procédé permettant d'ajuster spatialement les emplacements des centres de marqueurs (5) associés à un instrument médical (1) comportant un point d'intérêt (4), le procédé comprenant les étapes suivantes :
- mesurer les emplacements initiaux des centres par rapport au point d'intérêt (4),
- calculer les emplacements corrigés des centres par rapport au point d'intérêt (4) sur la base d'une géométrie prédéfinie de l'instrument médical (1), et
- ajuster les marqueurs (5) de telle sorte que les emplacements des centres des marqueurs (5) correspondent aux emplacements corrigés.

2. Procédé selon la revendication 1, dans lequel les marqueurs (5) sont des marqueurs réfléchissants plats et l'ajustement des marqueurs (5) consiste à rendre une partie d'au moins un marqueur (5) non réfléchissante.

3. Procédé selon la revendication 2, dans lequel le fait de rendre une partie d'un marqueur (5) non réfléchissante consiste à appliquer un revêtement non réfléchissant sur ladite partie du marqueur (5), à retirer ladite partie du marqueur (5) ou à désactiver ladite partie du marqueur (5).

4. Procédé selon l'une quelconque des revendications 1 à 3, dans lequel l'ajustement des marqueurs (5) consiste à modifier la position d'au moins un marqueur (5) par rapport à l'instrument médical (1).

5. Procédé selon la revendication 4, dans lequel la modification de la position d'un marqueur (5) par rapport à l'instrument médical implique le réglage d'un mécanisme de réglage mécanique.

6. Procédé selon l'une quelconque des revendications 4 et 5, dans lequel la modification de la position d'un marqueur (5) par rapport à l'instrument médical (1) consiste à adapter la position dudit marqueur (5) et à renforcer la liaison dudit marqueur (5) avec l'instrument médical (1).

7. Procédé selon l'une quelconque des revendications 1 à 6, dans lequel le calcul des emplacements corrigés des centres consiste à déterminer des emplacements optimisés des centres pour lesquels la distance entre un emplacement calculé du point d'intérêt (4) de l'instrument médical (1) et un emplacement prévu du point d'intérêt (4) de l'instrument médical (1) est minimisée, l'emplacement calculé du point d'intérêt (4) étant calculé à partir d'un ensemble d'emplacements candidats des centres et de données d'étalonnage génériques représentant la géométrie prédéfinie de l'instrument médical (1).

8. Procédé selon la revendication 7, dans lequel la géométrie prédéfinie de l'instrument médical (1) représente des emplacements prédéfinis des centres des marqueurs (5) et d'un point d'intérêt (4), et le calcul de l'emplacement calculé du point d'intérêt (4) implique de transformer la géométrie prédéfinie de telle sorte que les emplacements prédéfinis des centres soient alignés avec les emplacements candidats des centres, et d'utiliser l'emplacement du point d'intérêt (4) dans la géométrie prédéfinie transformée comme emplacement calculé du point d'intérêt (4).

9. Procédé selon la revendication 7 ou 8, dans lequel le calcul des emplacements corrigés des centres implique le calcul de plusieurs emplacements calculés du point d'intérêt (4) pour plusieurs ensembles d'emplacements candidats des centres, et les emplacements candidats donnant lieu à la plus petite distance entre l'emplacement calculé correspondant du point d'intérêt (4) et l'emplacement prévu du point d'intérêt (4) sont sélectionnés comme emplacements calculés des centres.

10. Procédé selon la revendication 9, dans lequel les multiples ensembles d'emplacements candidats sont sélectionnés à partir d'un espace de recherche limité aux emplacements réalisables des centres.

11. Procédé selon la revendication 7, dans lequel la géométrie prédéfinie de l'instrument médical (1) représente des emplacements prédéfinis des centres des marqueurs (5) et d'un point d'intérêt (4), et le calcul de l'emplacement calculé du point d'intérêt (4) comprend :
- la génération d'une pluralité de matrices de transformation (Mᵢ), comprenant une matrice de transformation pour chaque marqueur (5), une matrice de transformation représentant la transformation de l'emplacement prédéfini du centre du marqueur (5) en l'emplacement prédéfini du point d'intérêt (4),
- l'assemblage d'une matrice de transformation globale (*C̃*) à partir de la pluralité de matrices de transformation (Mᵢ), et
- la multiplication de la matrice de transformation (*C̃*) par un vecteur candidat (X_{c}) comprenant l'ensemble des emplacements candidats.

12. Programme informatique qui, lorsqu'il est exécuté sur un ordinateur, amène celui-ci à :
- acquérir des données de mesure d'un instrument médical (1) comportant une pluralité de marqueurs (5) et un point d'intérêt (4), les données de mesure représentant les emplacements initiaux des centres des marqueurs (5) par rapport au point d'intérêt (4), et
- calculer des données de correction représentant les emplacements corrigés des centres par rapport au point d'intérêt (4) sur la base d'une géométrie prédéfinie de l'instrument médical (1).

13. Programme informatique selon la revendication 12, dans lequel le programme amène l'ordinateur à fournir en outre des données de modification indiquant comment les marqueurs (5) doivent être adaptés de sorte que les emplacements des centres des marqueurs (5) correspondent aux emplacements corrigés.

14. Ordinateur sur lequel le programme informatique selon la revendication 12 ou 13 est stocké et/ou exécuté.

15. Support de stockage non transitoire lisible par ordinateur sur lequel est stocké le programme informatique selon la revendication 12 ou 13.
